# EUROPEAN PATENT APPLICATION

(11) **EP 2 266 576 A1**
(43) Date of publication of application: **29.12.2010**
(21) Application number: 10176770.5
(22) Date of filing: 15.06.1998
(51) Int. Cl.: A61K 31/64, A61K 31/44

(54) **TREATMENT OF DIABETES WITH THIAZOLIDINEDIONE AND SULPHONYLUREA**

(30) Priority: 18.06.1997 GB 9712854; 27.03.1998 GB 9806710
(62) Divisional of application: 06075523.8
(71) Applicant: SmithKline Beecham Limited, Middlesex TW8 9GS (GB)
(72) Inventor: Smith, Stephen Alistair, Harlow, Essex CM19 5AW (GB)
(74) Representative: West, Vivien

(57) **Abstract**

A method for the treatment of diabetes mellitus and conditions associated with diabetes mellitus in a mammal, which method comprises administering an effective non-toxic and pharmaceutically acceptable amount of an insulin sensitiser and an insulin secretagogue, to a mammal in need thereof.

## Description

This invention relates to a method of treatment, in particular to a method for the treatment of diabetes mellitus, especially non-insulin dependent diabetes (NIDDM) or Type II diabetes and conditions associated with diabetes mellitus.

Insulin secretagogues are compounds that promote increased secretion of insulin by the pancreatic beta cells.

The sulphonylureas are well known examples of insulin secretagogues. The sulphonylureas act as hypoglycaemic agents and are used in the treatment of NIDDM (or Type II diabetes). Examples of sulphonylureas include glibenclamide, glipizide, gliclazide, glimepiride, tolazamide and tolbutamide.

European Patent Application, Publication Number 0,306,228 relates to certain thiazolidinedione derivatives disclosed as having antihyperglycaemic and antihyperlipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter 'Compound (I)'). WO94/05659 discloses certain salts of Compound (I) including the maleate salt at example 1 thereof.

Compound (I) is an example of a class of anti-hyperglycaemic agents known as 'insulin sensitisers'. In particular Compound (I) is a thiazolidinedione insulin sensitiser.

European Patent Applications, Publication Numbers: 0008203, 0139421, 0032128,0428312,0489663,0155845,0257781,0208420,0177353,0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

Another series of compounds generally recognised as having insulin sensitiser activity are those typified by the compounds disclosed in International Patent Applications, Publication Numbers WO93/21166 and WO94/01420. These compounds are herein referred to as 'acyclic insulin sensitisers'. Other examples of acyclic insulin sensitisers are those disclosed in United States Patent Number 5232945 and International Patent Applications, Publication Numbers WO92/03425 and WO91/19702.

Examples of other insulin sensitisers are those disclosed in European Patent Application, Publication Number 0533933, Japanese Patent Application Publication Number 05271204 and United States Patent Number 5264451.

The above mentioned publications are incorporated herein by reference.

It is now surprisingly indicated that Compound (I) in combination with an insulin secretagogue provides a particularly beneficial effect on glycaemic control such combination is therefore particularly useful for the treatment of diabetes mellitus, especially Type II diabetes and conditions associated with diabetes mellitus. The treatment is also indicated to proceed with minimum side effects.

Accordingly, the invention provides a method for the treatment of diabetes mellitus, especially Type II diabetes and conditions associated with diabetes mellitus in a mammal such as a human, which method comprises administering an effective non-toxic and pharmaceutically acceptable amount of an insulin sensitiser, such as Compound (I), and an insulin secretagogue, to a mammal in need thereof.

In another aspect the invention provides an insulin sensitiser, such as Compound (I), together with an insulin secretagogue for use in a method for the treatment of diabetes mellitus, especially Type II diabetes and conditions associated with diabetes mellitus.

The method comprises either co-administration of an insulin sensitiser, such as Compound (I), and an insulin secretagogue or the sequential administration thereof.

Co-administration includes administration of a formulation which includes both an insulin sensitiser, such as Compound (I), and a biguanide antihyperglycaemic agent or the essentially simultaneous administration of separate formulations of each agent.

In another aspect the invention provides the use of an insulin sensitiser, such as Compound (I), and an insulin secretagogue for use in the manufacture of a composition for the treatment of diabetes mellitus, especially Type II diabetes and conditions associated with diabetes mellitus.

Suitable insulin secretagogues include sulphonylureas.

Suitable sulphonylureas include glibenclamide, glipizide, gliclazide, glimepiride, tolazamide and tolbutamide.

Further sulphonylureas include acetohexamide, carbutamide, chlorpropamide, glibornuride, gliquidone, glisentide, glisolamide, glisoxepide, glyclopyamide and glycylamide.

Further suitable insulin secretagogues include repaglinide

A suitable thiazolidinedione insulin sensitiser is Compound (I).

Other suitable thiazolidinedione insulin sensitisers include (+) -5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone), 5-[4-[(1-methylcyclohexyl)methoxy]benzyl] thiazolidine-2,4-dione (or ciglitazone), 5-[4-[2-(5-ethylpyridin-2-yl)ethoxy]benzyl] thiazolidine-2,4-dione (or pioglitazone) or 5-[(2-benzyl-2,3-dihydrobenzopyran)-5-ylmethyl)thiazolidine-2,4-dione (or englitazone).

In one particular aspect, the method comprises the administration of 2 to 12 mg of Compound (I), especially when administered per day.

Particularly, the method comprises the administration of 2 to 4, 4 to 8 or 8 to 12 mg of Compound (I) per day.

Particularly, the method comprises the administration of 2 to 4mg of Compound (I), especially when administered per day.

Particularly, the method comprises the administration of 4 to 8mg of Compound (I), especially when administered per day.

Particularly, the method comprises the administration of 8 to 12 mg of Compound (I), especially when administered per day.

Preferably, the method comprises the administration of 2 mg of Compound (I), especially when administered per day.

Preferably, the method comprises the administration of 4 mg of Compound (I), especially when administered per day.

Preferably, the method comprises the administration of 8 mg of Compound (I), especially when administered per day.

It will be understood that the insulin sensitiser, such as Compound (I) and the insulin secretagogue are each administered in a pharmaceutically acceptable form, including pharmaceutically acceptable derivatives such as pharmaceutically acceptable salts, esters and solvates thereof, as appropriate of the relevant pharmaceutically active agent. In certain instances herein the names used for the relevant insulin secretagogues may relate to a particular pharmaceutical form of the relevant active agent: It will be understood that all pharmaceutically acceptable forms of the active agents per se are encompassed by this invention.

Suitable pharmaceutically acceptable salted forms of Compound (I) include those described in EP 0306228 and WO94/05659. A preferred pharmaceutically acceptable salt is a maleate.

Suitable pharmaceutically acceptable solvated forms of Compound (I) include those described in EP 0306228 and WO94/05659, in particular hydrates.

Suitable pharmaceutically acceptable forms of the insulin sensitiser and the insulin secretagogue depend upon the particular secretagogue used but include known pharmaceutically acceptable forms of the particular secretagogue chosen. Such derivatives are found or are referred to in standard reference texts such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) or the above mentioned publications.

Compound (I) or, a pharmaceutically acceptable salt thereof, or a pharmaceutically acceptable solvate thereof, may be prepared using known methods, for example those disclosed in EP 0306228 and WO94/05659. The disclosures of EP 0306228 and WO94/05659 are incorporated herein by reference.

Compound (I) may exist in one of several tautomeric forms, all of which are encompassed by the term Compound (I) as individual tautomeric forms or as mixtures thereof. Compound (I) contains a chiral carbon atom, and hence can exist in up to two stereoisomeric forms, the term Compound (I) encompasses all of these isomeric forms whether as individual isomers or as mixtures of isomers, including racemates.

The insulin secretagogue of choice is prepared according to known methods, such methods are found or are referred to in standard reference texts, such as the British and US Pharmacopoeias, Remington's Pharmaceutical Sciences (Mack Publishing Co.), Martindale The Extra Pharmacopoeia (London, The Pharmaceutical Press) (for example see the 31 st Edition page 341 and pages cited therein) or the above mentioned publications.

When used herein the term 'conditions associated with diabetes' includes those conditions associated with diabetes mellitus itself and complications associated with diabetes mellitus.

'Conditions associated with diabetes mellitus itself' include hyperglycaemia, insulin resistance, including acquired insulin resistance and obesity. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

'Complications associated with diabetes mellitus' includes renal disease, especially renal disease associated with Type II diabetes, neuropathy and retinopathy.

Renal diseases associated with Type II diabetes include nephropathy, glomerulonephritis, glomerular sclerosis, nephrotic syndrome, hypertensive nephrosclerosis and end stage renal disease.

As used herein the term 'pharmaceutically acceptable' embraces both human and veterinary use: for example the term 'pharmaceutically acceptable' embraces a veterinarily acceptable compound.

For the avoidance of doubt, when reference is made herein to scalar amounts, including mg amounts, of Compound (I) in a pharmaceutically acceptable form, the scalar amount referred to is made in respect of Compound (I) *per se*: For example 2 mg of Compound (I) in the form of the maleate salt is that amount of maleate salt which contains 2 mg of Compound (I).

Diabetes mellitus is preferably Type II diabetes.

The particularly beneficial effect on glycaemic control provided by the treatment of the invention is indicated to be a synergistic effect relative to the control expected for the sum of the effects of the individual active agents.

Glycaemic control may be characterised using conventional methods, for example by measurement of a typically used index of glycaemic control such as fasting plasma glucose or glycosylated haemoglobin (Hb Alc). Such indices are determined using standard methodology, for example those described in: Tuescher A, Richterich, P., Schweiz. med. Wschr. 101 (1971), 345 and 390 and Frank P., 'Monitoring the Diabetic Patent with Glycosolated Hemoglobin Measurements', Clinical Products 1988.

In a preferred aspect, the dosage level of each of the active agents when used in accordance with the treatment of the invention will be less than would have been required from a purely additive effect upon glycaemic control.

There is also an indication that the treatment of the invention will effect an improvement, relative to the individual agents, in the levels of advanced glycosylation end products (AGEs), leptin and serum lipids including total cholesterol, HDL-cholesterol, LDL-cholesterol including improvements in the ratios thereof, in particular an improvement in serum lipids including total cholesterol, HDL-cholesterol, LDL-cholesterol including improvements in the ratios thereof.

In the method of the invention, the active medicaments are preferably administered in pharmaceutical composition form. As indicated above, such compositions can include both medicaments or one only of the medicaments.

Accordingly, in one aspect the present invention also provides a pharmaceutical composition comprising an insulin sensitiser, such as Compound (I) especially 2 to 12 mg thereof, an insulin secretagogue and a pharmaceutically acceptable carrier therefor.

Such compositions may be prepared by admixing an insulin sensitiser, such as Compound (I) especially 2 to 12 mg thereof, the insulin secretagogue and a pharmaceutically acceptable carrier therefor.

Usually the compositions are adapted for oral administration. However, they may be adapted for other modes of administration, for example parenteral administration, sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The compositions are preferably in a unit dosage form in an amount appropriate for the relevant daily dosage.

Suitable dosages including unit dosages of the Compound of formula (I) comprise 1, 2, 3, 4, 5, 6, 7, 8, 9, 10, 11 or 12 mg of Compound (I).

In the treatment the medicaments may be administered from 1 to 6 times a day, but most preferably 1 or 2 times per day.

Particular dosages of Compound (I) are 2mg/day, 4mg/day, including 2mg twice per day, and 8 mg/day, including 4mg twice per day.

Suitable dosages including unit dosages of the insulin sensitiser or the insulin secretagogue, such as the sulphonyl urea, include the known dosages including unit doses for these compounds as described or referred to in reference text such as the British and US Pharmacopoeias, Remington's Pharmaceutical Sciences (Mack Publishing Co.), Martindale The Extra Pharmacopoeia (London, The Pharmaceutical Press) (for example see the 3 1 st Edition page 341 and pages cited therein) or the above mentioned publications.

Thus: a typical daily dosage of glibenclamide is in the range of from 2.5 to 20 mg, for example 10mg twice per day or 20mg once per day; a typical daily dosage of glipizide is in the range of from 2.5 to 40 mg; a typical daily dosage of gliclazide is in the range of from 40 to 320 mg; a typical daily dosage of tolazamide is in the range of from 100 to 1000 mg; a typical daily dosage of tolbutamide is in the range of from 1000 to 3000 mg; a typical daily dosage of chlorpropamide is in the range of from 100 to 500 mg; and a typical daily dosage of gliquidone is in the range of from 15 to 180 mg.

An example of a treatment of the invention comprises the administration of 4 mg of Compound (I), for example taken as 2mg twice per day, and 20mg of glibenclamide, for example taken as 10mg twice per day.

Repaglinide may be taken in amounts, usually in the range of from 0.5mg to 4mg and usually with meals, up to a typical maximum daily dosage of 16mg per day.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agent can be dissolved in the vehicle. To enhance the stability, the composition can be frozen after filling into the vial and the water removed under vacuum. Parenteral suspensions are prepared in substantially the same manner, except that the Compound (I) suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions may contain from 0.1% to 99% by weight, preferably from 10-60% by weight, of the active material, depending upon the method of administration.

Composition may, if desired, be in the form of a pack accompanied by written or printed instructions for use.

The compositions are prepared and formulated according to conventional methods, such as those disclosed in standard reference texts, for example the British and US Pharmacopoeias, Remington's Pharmaceutical Sciences (Mack Publishing Co.), Martindale The Extra Pharmacopoeia (London, The Pharmaceutical Press) (for example see the 3 1 st Edition page 341 and pages cited therein) and Harry's Cosmeticology (Leonard Hill Books) or the above mentioned publications.

The present invention also provides a pharmaceutical composition comprising an insulin sensitiser, such as Compound (I) especially 2 to 12 mg thereof, an insulin secretagogue and a pharmaceutically acceptable carrier therefor, for use as an active therapeutic substance.

In particular, the present invention provides a pharmaceutical composition comprising an insulin sensitiser, such as Compound (I) especially 2 to 12 mg thereof, an insulin secretagogue and a pharmaceutically acceptable carrier therefor, for use in the treatment of diabetes mellitus, especially Type II diabetes and conditions associated with diabetes mellitus.

A range of 2 to 4mg includes a range of 2.1 to 4, 2.2 to 4, 2.3 to 4, 2.4 to 4, 2.5 to 4, 2.6 to 4, 2.7 to 4, 2.8 to 4, 2.9 to 4 or 3 to 4mg.

A range of 4 to 8mg includes a range of 4.1 to 8, 4.2 to 8, 4.3 to 8, 4.4 to 8, 4.5 to 8, 4.6 to 8, 4.7 to 8, 4.8 to 8, 4.9 to 8, 5 to 8, 6 to 8 or 7 to 8mg.

A range of 8 to 12 mg includes a range of 8.1 to 12, 8.2 to 12, 8.3 to 12, 8.4 to 12, 8.5 to 12, 8.6 to 12, 8.7 to 12, 8.8 to 12, 8.9 to 12, 9 to 12, 10 to 12 or 11 to 12mg.

No adverse toxicological effects have been established for the compositions or methods of the invention in the abovementioned dosage ranges.

The following example illustrate the invention but do not limit it in any way.

### COMPOSITIONS

### A Concentrate Preparation

Approximately two thirds of the lactose monohydrate is passed through a suitable screen and blended with the milled maleate salt of Compound (I).

Sodium starch glycollate, hydoxypropyl methylcellulose, microcrystalline cellulose and the remaining lactose are passed through a suitable screen and added to the mixture. Blending is then continued. The resulting mixture is then wet granulated with purified water. The wet granules are then screened, dried on a fluid bed drier and the dried granules are passed through a further screen and finally homogenised.

**% COMPOSITION OF GRANULAR CONCENTRATE**

| **Ingredient** | **Quantity (%)** |
|---|---|
| Milled Compound (I) as maleate salt | 13.25 (pure maleate salt) |
| Sodium Starch Glycollate | 5.00 |
| Hydoxypropyl Methylcellulose 2910 | 5.00 |
| Microcrystalline Cellulose | 20.0 |
| Lactose Monohydrate, regular grade | to 100 |
| Purified water | * |

| | |
|---|---|
| * Removed during processing. | |

### B Formulation of the concentrate into tablets.

The granules from above are placed into a tumble blender. Approximately two thirds of the lactose is screened and added to the blender. The microcrystalline cellulose, sodium starch glycollate, magnesium stearate and remaining lactose are screened and added to the blender and the mixture blended together. The resulting mix is then compressed on a rotary tablet press to a target weight of 150mg for the 1, 2 and 4mg tablets and to a target weight of 300mg for the 8mg tablets.

The tablet cores are then transferred to a tablet coating machine, pre-warmed with warm air (approximately 65°C) and film coated until the tablet weight has increased by 2.0% to 3.5%.

| | **Quantity (mg per Tablet)** | | | |
|---|---|---|---|---|
| **Tablet Strength** | **1.0mg** | **2.0m g** | **4.0mg** | **8.0mg** |
| **Active Ingredient:** | | | | |
| Compound (I) maleate Concentrate granules | 10.00 | 20.00 | 40.00 | 80.00 |
| **Other Ingredients:** | | | | |
| Sodium Starch Glycollate | 6.96 | 6.46 | 5.46 | 10.92 |
| Microcrystalline Cellulose | 27.85 | 25.85 | 21.85 | 43.70 |
| Lactose monohydrate | 104.44 | 96.94 | 81.94 | 163.88 |
| Magnesium Stearate | 0.75 | 0.75 | 0.75 | 1.50 |
| **Total Weight of Tablet Core** | 150.0 | 150.0 | 150.0 | 300.0 |
| Aqueous film coating material | 4.5 | 4.5 | 4.5 | 9.0 |
| **Total Weight of Film Coated Tablet** | 154.5 | 154.5 | 154.5 | 309.0 |

## Claims

1. A pharmaceutical composition comprising 5-[4-[2-(N-methyl-N-(2-pyridyl)amino)ethoxy]benzyl]thiazolidine-2,4-dione (Compound (I), a sulphonylurea and a pharmaceutically acceptable carrier therefor.

2. A composition according to claim 1, wherein the sulphonylurea is glibenclamide, glipizide, gliclazide, glimepiride, tolazamide or tolbutamide.

3. A composition according to claim 1 or claim 2, which comprises 2 to 12 mg of Compound (I).

4. A composition according to any one of claims 1 to 3, which comprises 2 to 4, 4 to 8 or 8 to 12 mg of Compound (I).

5. A composition according to claim 1 which comprises 2 to 4mg of Compound (I).

6. A composition according to claim 1, which comprises 4 to 8mg of Compound (I).

7. A composition according to claim 1, which comprises 8 to 12 mg of Compound (I).

8. A composition according to claim 1, which comprises 2 mg of Compound (I).

9. A composition according to claim 1, which comprises 4 mg of Compound (I).

10. A composition according to claim 1, which comprises 8 mg of Compound (I).

11. A pharmaceutical composition comprising 5-[4-[2-(N-methyl-N-(2-pyridyl)amino)ethoxy]benzyl]thiazolidine-2,4-dione (Compound (I), a sulphonylurea and a pharmaceutically acceptable carrier therefor, for use as an active therapeutic substance.

12. A pharmaceutical composition comprising 5-[4-[2-(N-methyl-N-(2-pyridyl)amino)ethoxy]benzyl]thiazolidine-2,4-dione (Compound (I)), a sulphonylurea and a pharmaceutically acceptable carrier therefor, for use in the treatment of diabetes mellitus and conditions associated with diabetes mellitus.

13. A composition according to any one of claims 1 to 12, wherein Compound (I) is present as a pharmaceutically acceptable derivative.

14. A composition according to claim 13, wherein the pharmaceutically acceptable derivative of Compound (I) is a salt.

15. A composition according to claim 13, wherein the pharmaceutically acceptable derivative of Compound (I) is a maleate salt.

16. A composition according to any one of claims 1 to 15, wherein the sulphonylurea is present as pharmaceutically acceptable derivative.
